# EUROPÄISCHE PATENTSCHRIFT

(11) **EP 1 750 781 B1**
(45) Veröffentlichungstag und Bekanntmachung des Hinweises auf die Patenterteilung: **29.12.2010**
(21) Anmeldenummer: 04735860.1
(22) Anmeldetag: 03.06.2004
(51) Int. Cl.: A61L 27/56, A61L 27/28

(54) **VORRICHTUNG ZUM IMPRÄGNIEREN EINES PORÖSEN KNOCHENERSATZMATERIALS**
DEVICE FOR IMPREGNATING A POROUS BONE REPLACEMENT MATERIAL
DISPOSITIF SERVANT A IMPREGNER UN MATERIAU POREUX DE SUBSTITUTION OSSEUSE

(43) Veröffentlichungstag der Anmeldung: 14.02.2007
(73) Patentinhaber: Synthes GmbH, 4436 Oberdorf (CH)
(72) Erfinder: HÖRGER, Flavio, CH-2502 Biel (CH); STOLL, Thierry, CH-2554 Meinisberg (CH)
(74) Vertreter: Lusuardi, Werther
(86) Internationale Anmeldenummer: PCT/CH2004/000335
(87) Internationale Veröffentlichungsnummer: WO 2005/118017

(56) Entgegenhaltungen:
- EP-A- 1 230 942
- WO-A2-99/59500
- DE-A1- 4 141 129
- US-A- 5 846 484
- PATENT ABSTRACTS OF JAPAN Bd. 2003, Nr. 05, 12. Mai 2003 (2003-05-12) & JP 2003 010301 A (NIPPON CERAPURE KK), 14. Januar 2003 (2003-01-14)

## Beschreibung

Die Erfindung bezieht sich auf eine Vorrichtung zum Imprägnieren eines porösen Knochenersatzmaterials gemäss dem Oberbegriff des Patentanspruchs 1.

Eine Vorrichtung zur Imprägnierung eines porösen, biokompatiblen Knochenersatzkörpers ist aus der US 6,049,026 MUSCHLER bekannt. Diese bekannte Vorrichtung umfasst eine Kammer zur Aufnahme des Knochenersatzkörpers sowie oberhalb der Kammer einen ersten Behälter zur Speicherung eines Imprägniermittels und unterhalb der Kammer einen zweiten Behälter zur Aufnahme des durch die Kammer mit dem Knochenersatzkörper fliessenden Imprägniermittels. Durch Öffnen eines ersten, zwischen dem ersten Behälter und der Kammer angeordneten Ventils fliesst das Imprägnierungsmittel in die Kammer mit dem Knochenersatzkörper. Sobald die Kammer gefüllt ist, wird ein zweites, zwischen der Kammer und dem zweiten Behälter angeordnetes Ventil geöffnet, so dass das Imprägniermittel durch eine unterhalb des Knochenersatzkörpers angeordnete Membran in den zweiten Behälter abfliessen kann. Nachteilig an dieser bekannten Vorrichtung ist, dass der Knochenersatzkörper in der Kammer nicht festgeklemmt werden kann, so dass der Knochenersatzkörper, bzw. das Implantat beispielsweise beim Transport durch Hin- und Herschütteln beschädigt oder gar zerbrechen könnte.

Hier will die Erfindung Abhilfe schaffen. Der Erfindung liegt die Aufgabe zugrunde, eine Vorrichtung zum Imprägnieren eines porösen Knochenersatzmaterials zu schaffen, bei welcher das Implantat, d.h. ein Körper aus Knochenersatzmaterial im Hohlraum gegen Beschädigung durch Hin- und Herschütteln beispielsweise beim Transport gesichert ist.

Die Erfindung löst die gestellte Aufgabe mit einer Vorrichtung zum Imprägnieren eines porösen Knochenersatzmaterials, welches die Merkmale des Anspruchs 1 aufweist. Die durch die erfindungsgemässe Vorrichtung erreichten Vorteile sind im wesentlichen darin zu sehen, dass dank der erfindungsgemässen Vorrichtung
- eine einzige Behältergrösse genügt, um verschieden grosse Implantate darin aufnehmen zu können; und
- ein in den Hohlraum des Behälters eingelegtes Implantat gehalten und zentriert wird und gegen Beschädigung oder Zerbrechen beispielsweise bei einem Hin- und Herschütteln oder beim Transport des Behälters geschützt ist.

Die elastischen Mittel können vorzugsweise in Form eines Balges oder einer Feder ausgebildet sein. Die elastischen Mittel können auch ein mehrere, bezüglich der Zentralachse helix- oder spiralförmig angeordnete, elastische Stege umfassendes, zur Zentralachse koaxial angeordnetes Federelement beinhalten. Die Ausgestaltung des Federelementes mit solchen Stegen ermöglicht das Anbringen von grossen Durchgangsöffnungen für das Imprägniermittel, so dass ein geringer Widerstand beim Durchströmen des Federelementes entsteht. Ein weiterer Vorteil besteht darin, dass die Haltekraft auf das Implantat gleichmässig ist und das Implantat noch zusätzlich zentriert wird.

Die Stege können gegen den Deckel gerichtete, obere Enden aufweisen, welche in eine mit Perforationen versehene und zur Zentralachse konzentrisch angeordnete, zentrale Platte münden, deren orthogonal zur Zentralachse gemessener Durchmesser d zwischen 0,001 % und 99,999 % und vorzugsweise zwischen 0,1 % und 99,9 % des zur Zentralachse orthogonalen Durchmessers D des Hohlraums betragen kann. Der Durchmesser d kann zwischen 1 % und 99 %, vorzugsweise zwischen 5 % und 95 % des Durchmessers D betragen. Typischerweise beträgt der Durchmesser d zwischen 10 % und 90 %, vorzugsweise zwischen 20 % und 80 % des Durchmessers D. Vorteilhafterweise beträgt der Durchmesser d zwischen 25 % und 75 %, vorzugsweise zwischen 30 % und 40 % des Durchmessers D. Der relativ zum Durchmesser des Hohlraums kleine Durchmesser der zentralen Platte ermöglicht, dass nur ein geringer Anteil der Oberfläche des Implantates dem Imprägniermittel nicht ausgesetzt ist. Ein weiterer Vorteil besteht darin, dass die Haltekraft auf das Implantat gleichmässig ist und das Implantat noch zusätzlich zentriert wird.

Bei einer weiteren Ausführungsform weisen die Stege untere Enden auf, welche in einen Aussenring des Federelementes münden. Der Aussenring kann dabei quer zur Zentralachse spielfrei im Hohlraum gelagert sein. Die elastischen Mittel sind somit nach dem Verschrauben des Deckels durch das gegen das Implantat drückende Federelement und durch die Spielfreiheit im Hohlraum fixiert. Weitere Vorteile liegen darin begründet, dass die Form der Dichtung kein Abkippen aus der Achse erlaubt, und dass das Totvolumen des unteren Aussenrings verringert wird, so dass weniger Blut für die Perfusion verwendet werden muss.

Bei einer weiteren Ausführungsform ist zwischen Behälter und dem Deckel eine Dichtung angeordnet. Dadurch bleibt der Behälter auch bei einem Vakuum dicht.

Bei einer weiteren Ausführungsform sind das Federelement und die Dichtung einstückig ausgebildet. Dadurch ergibt sich ein einfaches Zusammenfügen der Vorrichtung und die Dichtung kann nicht verloren gehen.

Bei einer weiteren Ausführungsform ist die parallel zur Zentralachse gemessene Höhe H des Hohlraumes durch die parallel zur Zentralachse gemessenen Höhen des Aussenringes und der Dichtung definiert. Dadurch können beispielsweise verschiedene Dichtungen oder verschiedene Federelemente zur Definition der Hohlraumhöhe eingesetzt werden. Ein weiterer Vorteil liegt in den verringerten Produktions- und Verpackungskosten, da ein einziger Behälter für eine Vielzahl von unterschiedlich grossen Implantaten verwendet werden kann.

Alternativ kann die parallel zur Zentralachse gemessene Höhe durch verschieden hohe Innenteile, vorzugsweise durch den Deckel definiert sein.

Bei einer weiteren Ausführungsform weisen die elastischen Mittel - parallel zur Zentralachse gemessen - im unbelasteten Zustand eine Höhe h auf und sind um ein Mass Δh axial komprimierbar, wobei das Verhältnis Δh : h zwischen 0,001 % und 99,999 %, vorzugsweise zwischen 0,01 % und 99,99 % liegt. Typischerweise kann das Verhältnis Δh : h zwischen 0,1 % und 99,9 %, vorzugsweise zwischen 1 % und 99 % betragen. Vorteilhafterweise liegt das Verhältnis Δh : h zwischen 5 % und 95 %, vorzugsweise zwischen 10 % und 90 %. Die elastischen Mittel sind dadurch in einem hohen Masse komprimierbar, so dass Implantate mit unterschiedlicher Grösse einsetzbar sind.

Bei einer weiteren Ausführungsform ist der Behälter kreiszylindrisch ausgebildet und weist ein im Hohlraum koaxial angeordnetes Innengewinde auf, wobei der Deckel ein zum Innengewinde komplementäres Aussengewinde umfasst.

Vorteilhafterweise enthält der Behälter ein poröses Knochenersatzmaterial, dessen Gesamtvolumen v kleiner ist als das Volumen V des Hohlraumes (13). Das Knochenersatzmaterial kann in Form eines Blocks, vorzugsweise in Form eines Würfels, Zylinders, Hohlzylinders, Scheibe, Keils, Kegels, Kegelstumpfes oder einer Kugel vorliegen. Das Knochenersatzmaterial kann derart in einem Implantat untergebracht sein, dass es mindestens teilweise mit der Oberfläche des Implantates kommuniziert.

Das Implantat kann aus einer der folgenden Materialgruppen ausgewählt werden:
A) Metall und/oder
B) Kunststoff und/oder
C) Nichtmetall, vorzugsweise Glas und/ oder
D) unterschiedlich dichte Keramiken und/oder
E) zusammengesetzte Keramiken.

Die elastischen Mittel können entweder symmetrisch oder asymmetrisch zur Zentralachse angeordnet sein. Je nach Ausbildung oder Form des Implantates bietet eine symmetrische oder eine asymmetrische Konstruktion einen besseren Halt für das Implantat.

Der Boden des Behälters kann mit einem zentral oder dezentral durchbohrten Anschlusstück versehen sein, welcher eine der beiden Öffnungen bildet. Auch aussen an der Deckplatte des Deckels kann ein zentral oder dezentral durchbohrtes Anschlussstück angeordnet sein, welches eine der beiden Öffnungen bildet.

Das Knochenersatzmaterial kann in Form eines Blocks, vorzugsweise in Form eines Würfels, Zylinders, Hohlzylinders, Scheibe, Keils, Kegels, Kegelstumpfes oder einer Kugel vorliegen.

Das Imprägniermittel kann osteoinduktive und/oder osteogene Substanzen, insbesondere Körperzellen, Knochenmark oder Knochenmarkbestandteilen, Blut oder Blutbestandteilen oder eine Kombination davon umfassen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Schnitt durch eine Ausführungsform der erfindungsgemässen Vorrichtung;
Fig. 2 eine Aufsicht auf die in Fig. 1 dargestellte Ausführungsform der erfindungsgemässen Vorrichtung;
Fig. 3 eine perspektivische Ansicht eines elastischen Mittelteils der in den Fig. 1 und 2 dargestellten Ausführungsform der erfindungsgemässen Vorrichtung;
Fig. 4 einen Schnitt durch das in Fig. 3 dargestellte elastische Mittelteil;
Fig. 5 eine Ansicht eines durch den Kreis A in Fig. 1 markierten Anschlussstückes;
Fig. 6 eine Aufsicht auf das in Fig. 5 dargestellte Anschlussstück; und
Fig. 7 eine perspektivische Ansicht einer Ausführungsform der erfindungsgemässen Vorrichtung und einer Spritze.

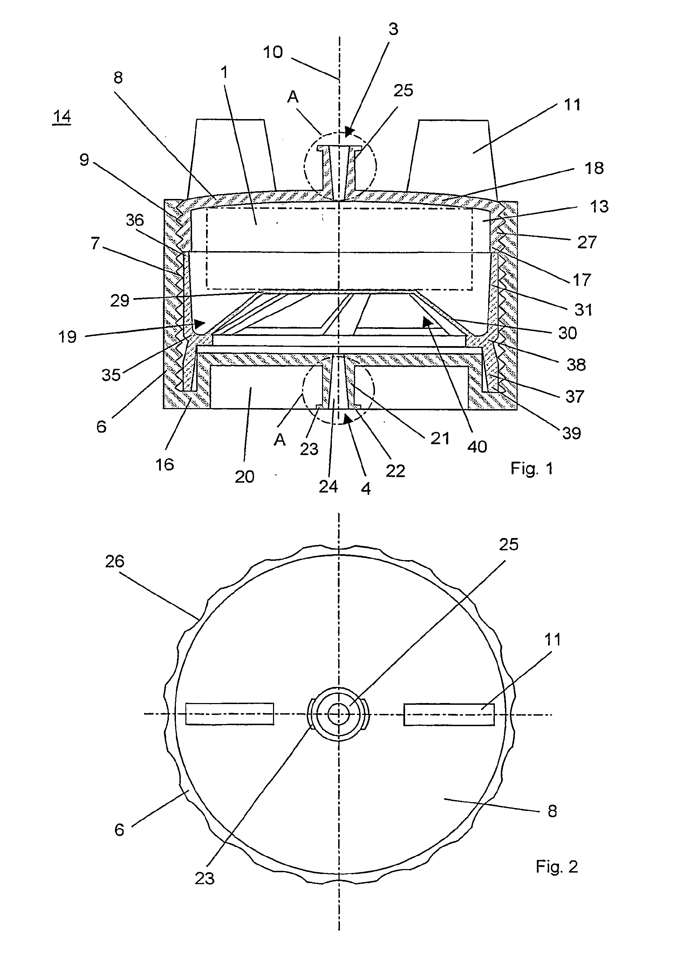

In Fig. 1 ist eine Ausführungsform der erfindungsgemässen Vorrichtung dargestellt, welche einen zur Zentralachse 10 koaxialen, kreiszylindrischen Behälter 6 mit einem koaxial auf diesem befestigbaren Deckel 8 umfasst. Der Behälter 6 weist in seinem Hohlraum 13 ein zur Zentralachse 10 koaxiales Innengewinde 7 auf, so dass der Deckel 8, welcher auf seiner seitlichen Mantelfläche ein zum Innengewinde 7 komplementäres Aussengewinde 9 aufweist, mit dem Behälter 6 lösbar verbindbar ist. Das Innengewinde 7 erstreckt sich über die gesamte axiale Länge des Hohlraumes 13 im Behälter 6 (alternativ könnte auch ein mehrgängiges Gewinde nur bis zur Hälfte des Behälters 6 verwendet werden). Das Aussengewinde 9 an der seitlichen Mantelfläche des Deckels 8 erstreckt sich über die gesamte axiale Länge des Deckels 8. Im Hohlraum 13 angeordnet sind die elastischen Mittel 19, welche ein insbesondere axial elastisches Federelement 30 und eine damit einstückige Dichtung 31 umfassen.

Die hohlzylindrische Dichtung 31 erstreckt sich parallel zur Zentralachse 10 zwischen dem oberen Ende 38 des zum Federelement 30 zählenden Aussenrings 37 und dem unteren Ende 17 des Deckels 8. Das untere Ende 39 des Aussenringes 37 steht auf dem Boden 16 des Behälters 6 auf, während das obere Ende 38 des Aussenringes 37 mit dem unteren Ende 35 der Dichtung 31 verbunden ist. Der Aussenring 37 ist im Aussendurchmesser passend zum Innendurchmesser des Behälters 6 ausgestaltet, so dass die elastischen Mittel 19 gegen Verschiebungen quer zur Zentralachse 10 gesichert sind.

Der Deckel 8 ist soweit in den Hohlraum 13 des Behälter 6 einschraubbar bis das untere Ende 17 des Deckels 8 auf dem oberen Ende 36 der Dichtung 31 aufsteht. Das Innengewinde 7 sowie das Aussengewinde 9 sind als mehrgängige Gewinde ausgestaltet. Der Boden 16 des Behälters 6 ist mit einer zur Zentralachse 10 konzentrischen Vertiefung 20 versehen, wo konzentrisch zur Zentralachse 10 ein zentral durchbohrtes Anschlusstück 21 angeordnet ist. Die Zentralbohrung 24 des Anschlusstückes 21 bildet die Öffnung 4. Analog dazu ist aussen an der Deckplatte 18 des Deckels 8 ein zweites, konzentrisch zur Zentralachse 10 durchbohrtes Anschlussstück 25 angeordnet. Die Anschlussstücke 21 und 25 sind identisch ausgestaltet (alternativ könnten die beiden Anschlussstücke 21 und 25 auch unterschiedlich ausgestaltet werden).

Ein in den Hohlraum 13 des Behälters 6 eingelegtes Stück Knochenersatzmaterial 1 wird beim Einschrauben des Deckels 8 in den Behälter 6 gegen das obere Ende 29 des Federelementes 30 gepresst, so dass das Federelement 30 axial um ein Mass Δh komprimiert wird. Durch die Elastizität des Federelementes 30 wird der Körper aus Knochenersatzmaterial 1 zwischen dem oberen Ende 29 des Federelementes 30 und der Deckelplatte 18 des Deckels 8 eingespannt.

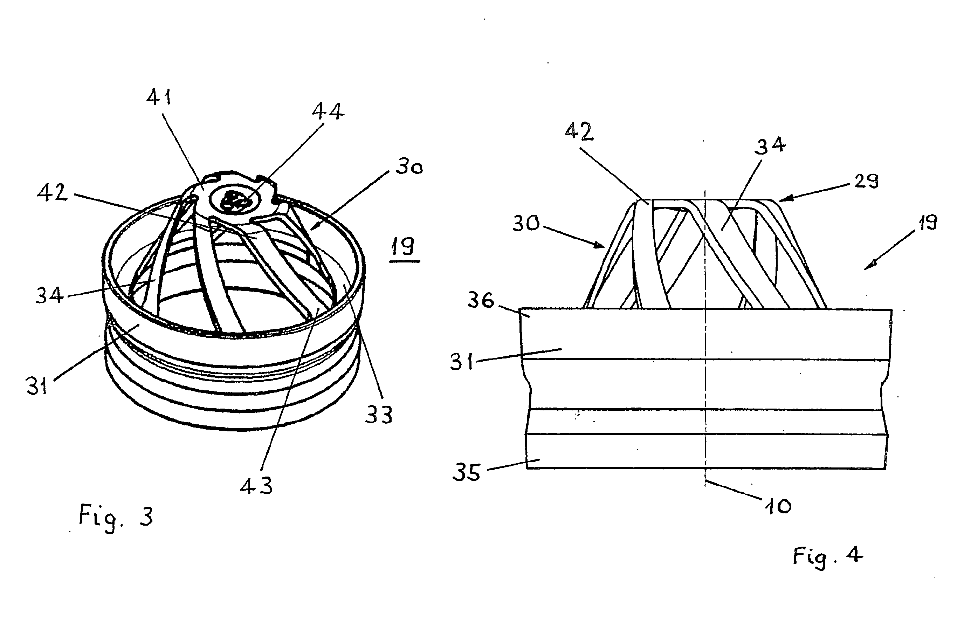

In den Fig. 3 und 4 ist eine Ausführungsform der elastischen Mittel 19 dargestellt, welche ein Federelement 30 mit einem Aussenring 37 und einem am oberen Ende 38 des Aussenringes 37 angeordneten kegelstumpfartigen Segment 40. Dieses kegelstumpfartige Segment 40 umfasst Stege 34, deren obere Enden 42 in eine am oberen Ende 29 des Federelementes 30 in eine zur Zentralachse 10 konzentrisch angeordnete, zentrale Platte 41 mit Perforierungen 44 münden und deren untere Enden 43 mit dem Aussenring 37 verbunden sind. Das kegelstumpfartige Segment 40 ist gegen das obere Ende 29 des Federelementes 30 verjüngt ausgebildet, wobei die Stege 34 spiralförmig um die Zentralachse 10 verlaufen.

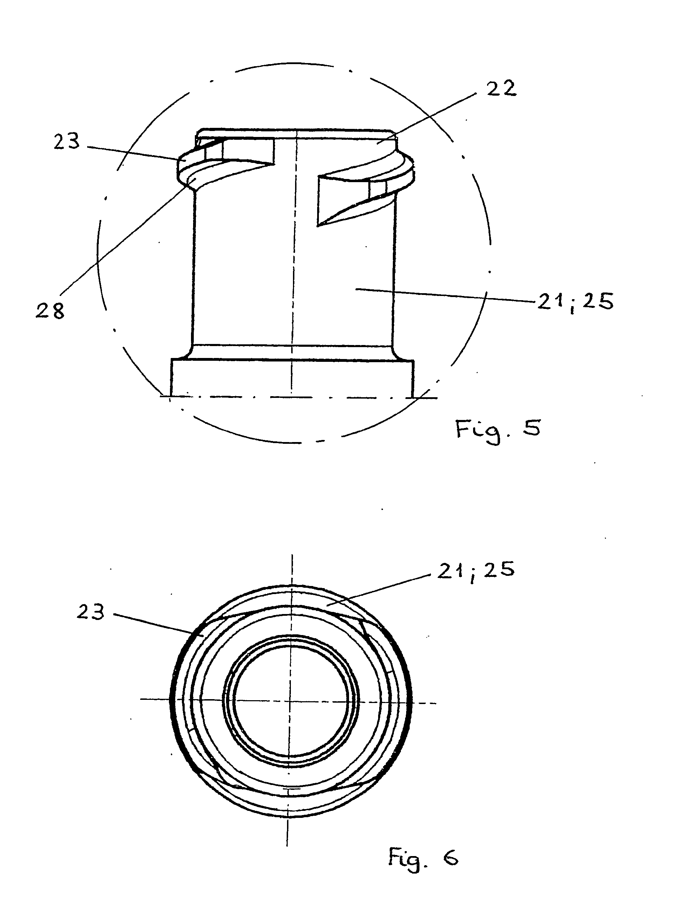

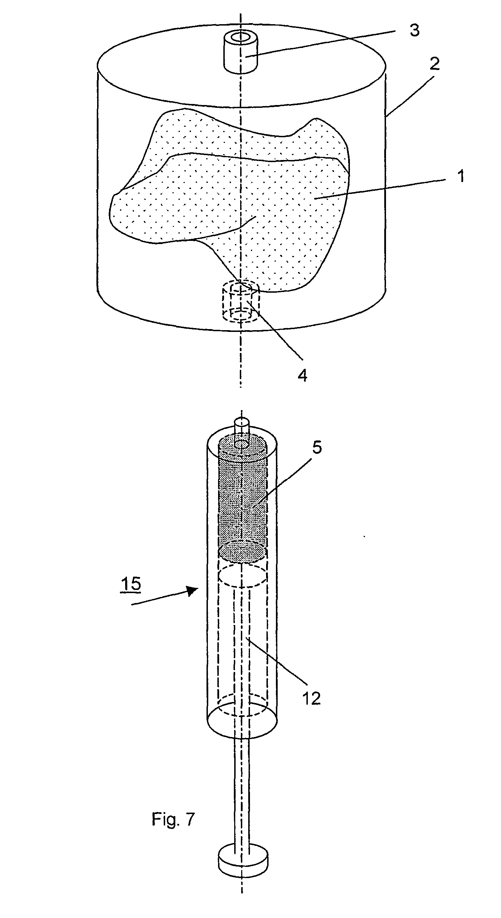

In den Fig. 5 und 6 dargestellt sind die als männliche Luer-Verbindungen identisch ausgebildeten Anschlussstücke 21;25. Jedes der Anschlussstücke 21;25 umfasst an seinem freien Ende 22 zwei radial vorstehende Nocken 23, welche durch je zwei einander diametral gegenüberliegende Segmente eines Gewindeganges 28 gebildet werden. Eine an ihrer Öffnung zum Anschlusstück 21 als komplementär weibliche Luer-Verbindung ausgestaltet Standardspritze 15 (Fig. 7) ist über jeder der Anschlussstücke 21 ;25 schraubbar(Luer-Verbindung). Die Anschlüsse können so gewählt werden, dass verschiedene Adapter luftdicht angeschlossen werden können.

Durch die Anordnung (Fig. 1) des Anschlusstückes 21 in der Vertiefung 20 derart, dass das Anschlusstück 21 nicht über den Boden 16 des Behälters 6 vorsteht, wird erreicht, dass der Behälter 6 auf den Boden gestellt werden kann, ohne zu kippen Zur Erleichterung des Verschraubens oder Aufschraubens des Deckels 8 sind auf der Aussenseite der Deckplatte 18 am Deckel 8 zwei axial vorstehende Erhebungen 11 und an der äusseren Mantelfläche des Behälters 6 axial verlaufende Rillen 26 angebracht (Fig. 2).

Fig. 7 zeigt einen Behälter 6 mit einem im Hohlraum eingeschlossenen, aus Knochenersatzmaterial 1 bestehenden Körper. Der Behälter 6 hat zwei Öffnungen 3;4, welche mit einer Spritze 15 luftdicht verbindbar sind. Als Imprägniermittel 5 kann die Spritze 15 in ihrem Hohlraum osteoinduktive und/oder osteogene Substanzen, insbesondere Körperzellen, Knochenmark und/oder Knochenmarkbestandteile, Blut und/oder Blutbestandteile enthalten. Der hier dargestellte Behälter 6 dient zusammen mit einer Spritze 15 dazu, das die folgenden Schritte umfassende erfindungsgemässe Verfahren auszuführen:
A) Eine mit Imprägniermittel 5 gefüllte Spritze 15 wird vorzugsweise an die untere der beiden Öffnungen 4 des Behälters 6 angeschlossen. Die obere Öffnung 3 bleibt unverschlossen. Beide Öffnungen 3;4 sind als Luer-Öffnungen mit einem konisch durchbohrten, mit dem Behälter 6 verbundenen Anschlussstück 21;25 ausgestaltet (Fig. 5 und 6). Das Imprägniermittel 5 wird nun mittels des Kolbens 12 durch die untere Öffnung 4 in den Behälter 6 eingespritzt, so dass der Block aus porösem Knochenersatzmaterial 1 vom Imprägniermittel 5 umgeben wird und in diesem teilweise, vorzugsweise ganz untertaucht;
B) Nun wird die obere Öffnung 3 verschlossen;
C) Danach wird der Kolben 12 der Spritze 5 wieder zurückgezogen, so dass in dem Behälter 6 ein Unterdruck oder Vakuum entsteht. Durch das Vakuum wird nun die in den Poren des Knochenersatzmaterials 1 vorhandene Luft zum Expandieren gebracht, so dass sie aus den Poren in das umgebende Imprägniermittel 5 austritt. Da es sich um ein geschlossenes System handelt, wird das Imprägniermittel 5 nur teilweise durch die Kolbenbewegung abgesaugt. Dies ist auch nur möglich, wenn noch Luft in dem Behälter 6 enthalten ist. Bei einer grossen Anzahl von Imprägniermitteln 5 kommt hinzu, dass sie adhärente Eigenschaften aufweisen, somit auf der Oberfläche des Knochenersatzmaterials 1 anhaften und durch die Kolbenbewegung nicht abgesaugt werden;
D) In einem nächsten Schritt wird der Kolben 12 der Spritze 5 wieder in die ursprüngliche Position gedrückt, so dass das Vakuum im Behälter 6 aufgehoben wird. Der vom Imprägniermittel 5 umgebene Block aus Knochenersatzmaterial 1 nimmt nun in seinen Poren natürlich keine Luft sondern Imprägniermittel 5 auf, so dass eine Imprägnierung des Knochenersatzmaterials 1 stattfindet. Die mittels der Spritze 5 durchführbare Evakuierung/Aufhebung des Vakuums im Behälter kann mehrere Male wiederholt werden, um den Imprägnierungsgrad zu erhöhen. Das Imprägniermittel 5 wird durch dessen Adhäsion und durch die Kapillarwirkung der Struktur des porösen Knochenersatzmaterials 1 eher ins Innere aufgenommen als Luft.

Eine andere Ausführung des Verfahrens besteht darin, dass nachdem die erste, mit Imprägniermittel 5 gefüllte Spritze 15 an eine der zwei Öffnungen 3;4 angekuppelt worden ist, eine zweite, ungefüllte Spritze (nicht gezeichnet) an die andere Öffnung 3;4 angekuppelt wird, und durch Zurückziehen des Kolbens der Hohlraum des Behälters 6 evakuiert wird und zugleich das Imprägniermittel 5 durch den entstehenden Unterdruck aus dem Hohlraum in der Spritze 15 in den Hohlraum des Behälters 6 gesaugt wird. Die Luft in den Poren des Knochenersatzmateriales 1 tritt aus den Poren aus. Anschliessend wird die Luft durch Einschieben des Kolbens der zweiten Spritze wieder in den Behälter 6 gefördert, so dass das Vakuum im Hohlraum des Behälters 6 wieder aufgehoben wird und das Imprägniermittel 5 in die Poren des Knochenersatzmaterials eindringen kann. Falls erforderlich kann nun der Kolben einer der Spritzen zurückgezogen werden und der Behälter 6 erneut evakuiert werden. Die Verfahrensschritte des Evakuierens und des Aufhebens des Vakuums können auf diese Weise einfach wiederholt werden, bis die Poren im Knochenersatzmaterial 1 ausreichend entlüftet und mit Imprägniermittel 5 gefüllt sind.

Wiederum eine andere Ausführung des Verfahrens besteht darin, dass die zweite Spritze (nicht gezeichnet) zur Vergrösserung des Vakuums angewendet wird. Diese zweite Spritze kann - da sie nicht mit Imprägniermittel 5 gefüllt ist - ein erheblich grösseres Volumen aufweisen als die erste, mit Imprägniermittel 5 gefüllte Spritze 15.

## Patentansprüche

1. Vorrichtung (14) zur Imprägnierung eines porösen Knochenersatzmaterials (1) mit einem Imprägniermittel (5), mit
A) einem eine Zentralachse (10) und einen Hohlraum (13) aufweisenden Behälter (6) mit einem Deckel (8); wobei
B) der Behälter (2) zwei verschliessbare Öffnungen (3,4) aufweist,
**dadurch gekennzeichnet, dass**
C) der Behälter (6) im Hohlraum (13) angeordnete, elastische Mittel (19) umfasst, mittels welcher auf ein im Hohlraum (13) eingelegtes Knochenersatzmaterial (1) eine Klemmkraft ausübbar ist;
D) der Behälter (6) so ausgestaltet ist, dass er evakuiert werden kann; und
E) der Behälter (6) in seinem Hohlraum (13) ein zur Zentralachse (10) koaxiales Innengewinde (7) aufweist, so dass der Deckel (8), welcher auf seiner seitlichen Mantelfläche ein zum Innengewinde (7) komplementäres Aussengewinde (9) aufweist, mit dem Behälter (6) lösbar verbindbar ist.

2. Vorrichtung (14) nach Anspruch 1, **dadurch gekennzeichnet, dass** die elastischen Mittel (19) in Form eines Balges oder einer Feder ausgebildet sind.

3. Vorrichtung (14) nach Anspruch 1, **dadurch gekennzeichnet, dass** die elastischen Mittel (19) ein mehrere bezüglich der Zentralachse (10) helix- oder spiralförmig angeordnete, elastische Stege (34) umfassendes, zur Zentralachse (10) koaxial angeordnetes Federelement (30) beinhalten.

4. Vorrichtung (14) nach Anspruch 3, **dadurch gekennzeichnet, dass** die Stege (34) gegen den Deckel (8) gerichtete, obere Enden (42) haben, welche in eine mit Perforationen (44) versehene und zur Zentralachse (10) konzentrisch angeordnete, zentrale Platte (41) münden, deren orthogonal zur Zentralachse (10) gemessener Durchmesser d zwischen 0,001 % und 99,999 %, vorzugsweise zwischen 0,1 % und 99,9 % des zur Zentralachse (10) orthogonalen Durchmessers D des Hohlraums (13) beträgt.

5. Vorrichtung (14) nach Anspruch 4, **dadurch gekennzeichnet, dass** Durchmesser d zwischen 1 % und 99 %, vorzugsweise zwischen 5 % und 95 % des Durchmessers D beträgt.

6. Vorrichtung (14) nach Anspruch 5, **dadurch gekennzeichnet, dass** Durchmesser d zwischen 10 % und 90 %, vorzugsweise zwischen 20 % und 80 % des Durchmessers D beträgt.

7. Vorrichtung (14) nach Anspruch 6, **dadurch gekennzeichnet, dass** Durchmesser d zwischen 25 % und 75 %, vorzugsweise zwischen 30 % und 40 % des Durchmessers D beträgt.

8. Vorrichtung (14) nach einem der Ansprüche 3 bis 7, **dadurch gekennzeichnet, dass** die Stege (34) untere Enden (43) aufweisen, welche in einen Aussenring (37) des Federelementes (30) münden.

9. Vorrichtung (14) nach Anspruch 8, **dadurch gekennzeichnet, dass** der Aussenring (37) quer zur Zentralachse (10) spielfrei im Hohlraum (13) gelagert ist.

10. Vorrichtung (14) nach einem der Ansprüche 1 bis 9, **dadurch gekennzeichnet, dass** zwischen Behälter (6) und dem Deckel (8) eine Dichtung (31) angeordnet ist.

11. Vorrichtung (14) nach Anspruch 10, **dadurch gekennzeichnet, dass** das Federelement (30) und die Dichtung (31) einstückig sind.

12. Vorrichtung (14) nach Anspruch 10 oder 11, **dadurch gekennzeichnet, dass** die parallel zur Zentralachse (10) gemessene Höhe H des Hohlraumes (13) durch die parallel zur Zentralachse (10) gemessenen Höhen des Aussenringes (37) und der Dichtung (31) definiert ist.

13. Vorrichtung (14) nach Anspruch 10 oder 11, **dadurch gekennzeichnet, dass** die parallel zur Zentralachse (10) gemessene Höhe durch verschieden hohe Innenteile, vorzugsweise durch den Deckel (8) definiert ist.

14. Vorrichtung (14) nach einem der Ansprüche 1 bis 13, **dadurch gekennzeichnet, dass** die elastischen Mittel (19) parallel zur Zentralachse (10) gemessen im unbelasteten Zustand eine Höhe h aufweisen und um ein Mass Δh axial komprimierbar sind, und dass das Verhältnis Δh : h zwischen 0,001 % und 99,999 %, vorzugsweise zwischen 0,01 % und 99,99 % beträgt.

15. Vorrichtung (14) nach Anspruch 14, **dadurch gekennzeichnet, dass** Verhältnis Δh : h zwischen 0,1 % und 99,9 %, vorzugsweise zwischen 1 % und 99 % liegt.

16. Vorrichtung (14) nach Anspruch 15, **dadurch gekennzeichnet, dass** Verhältnis Δh : h zwischen 5 % und 95 %, vorzugsweise zwischen 10 % und 90 % liegt.

17. Vorrichtung (14) nach einem der Ansprüche 1 bis 16, **dadurch gekennzeichnet, dass** der Behälter (6) kreiszylindrisch ausgebildet ist, ein im Hohlraum (13) koaxial angeordnetes Innengewinde (7) aufweist und dass der Deckel (8) ein zum Innengewinde (7) komplementäres Aussengewinde (9) umfasst.

18. Vorrichtung (14) nach einem der Ansprüche 1 bis 17, **dadurch gekennzeichnet, dass** der Behälter (6) ein poröses Knochenersatzmaterial (1) enthält, dessen Gesamtvolumen v kleiner als das Volumen V des Hohlraumes (13) ist.

19. Vorrichtung (14) nach Anspruch 18, **dadurch gekennzeichnet, dass** das Knochenersatzmaterial (1) in Form eines Blocks, vorzugsweise in Form eines Würfels, Zylinders, Hohlzylinders, Scheibe, Keils, Kegels, Kegelstumpfes oder einer Kugel vorliegt.

20. Vorrichtung nach Anspruch 18 oder 19, **dadurch gekennzeichnet, dass** das Knochenersatzmaterial (1) derart in einem Implantat untergebracht ist, dass es mindestens teilweise mit der Oberfläche des Implantates kommuniziert.

21. Vorrichtung nach Anspruch 20, **dadurch gekennzeichnet, dass** das Implantat aus einer der folgenden Materialgruppen ausgewählt ist:
A) Metall und/oder
B) Kunststoff und/oder
C) Nichtmetall, vorzugsweise Glas und/ oder
D) unterschiedlich dichte Keramiken und/oder
E) zusammengesetzte Keramiken.

22. Vorrichtung nach einem der Ansprüche 1 bis 21, **dadurch gekennzeichnet, dass** die elastischen Mittel (19) symmetrisch zur Zentralachse (10) angeordnet sind.

23. Vorrichtung nach einem der Ansprüche 1 bis 21, **dadurch gekennzeichnet, dass** die elastischen Mittel (19) asymmetrisch zur Zentralachse (10) angeordnet sind.

24. Vorrichtung nach einem der Ansprüche 1 bis 23, **dadurch gekennzeichnet, dass** der Boden (16) des Behälters (6) mit einem zentral durchbohrten Anschlusstück (21) versehen ist, welches eine der beiden Öffnungen (4) bildet.

25. Vorrichtung nach einem der Ansprüche 1 bis 23, **dadurch gekennzeichnet, dass** der Boden (16) des Behälters (6) mit einem dezentral durchbohrten Anschlusstück (21) versehen ist, welches eine der beiden Öffnungen (4) bildet.

26. Vorrichtung nach einem der Ansprüche 1 bis 25, **dadurch gekennzeichnet, dass** aussen an der Deckplatte (18) des Deckels (8) ein zentral oder dezentral durchbohrtes Anschlussstück (25) angeordnet ist, welches eine der beiden Öffnungen (3) bildet.

27. Verwendung der Vorrichtung nach einem der Ansprüche 1 bis 26 zur Imprägnierung eines porösen Knochenersatzmaterials (1) mit einem Imprägniermittel (5).

## Claims

1. Device (14) for impregnating a porous bone replacement material (1) with an impregnation agent (5), with
A) a container (6) with a lid (8); said container (6) presenting a central axis (10) and a cavity (13), where
B) the container (2) presents two closable openings (3, 4),
**characterized in that**
C) the container (6) comprises elastic means (19) arranged inside the cavity (13), by means of which a bone replacement material (1) introduced into the cavity (13) can be subjected to a clamping force;
D) the container (6) is configured in such a way that it can be evacuated; and
E) the container (6) presents inside its cavity (13) an inner thread (7) set up coaxially to the central axis (10), so that the lid (8), which has on its lateral mantle surface an outer thread (9) complementary to the inner thread (7), can be connected to the container (6) in a detachable manner.

2. Device (14) according to claim 1, **characterized in that** the elastic means (19) are configured like a bellows or a spring.

3. Device (14) according to claim 1, **characterized in that** the elastic means (19) contain a spring element (30) coaxially arranged with respect to the central axis (10) and comprising several elastic webs (34) which are helically or spirally arranged with respect to the central axis (10).

4. Device (14) according to claim 3, **characterized in that** the webs (34) have upper ends turned toward the lid (8), which run into a central plate (41) fitted with perforations (44) and arranged concentrically to the central axis (10), whose diameter d measured orthogonally to the central axis (10) amounts to between 0.001% and 99.999%, and preferably to between 0.1 % and 99.9% of the diameter D, measured orthogonally to the central axis (10) of the cavity (13).

5. Device (14) according to claim 4, **characterized in that** the diameter d is between 1% and 99%, and preferably between 5% and 95% of the diameter D.

6. Device (14) according to claim 5, **characterized in that** the diameter d is between 10% and 90%, and preferably between 20% and 80% of the diameter D.

7. Device (14) according to claim 6 **characterized in that** the diameter d is between 25% and 75%, and preferably between 30% and 40% of the diameter D.

8. Device (14) according to one of the claims from 3 to 7, claim 4 **characterized in that** the webs (34) present lower ends (43) which run into an outer ring (37) of the spring element (30).

9. Device (14) according to claim 8, **characterized in that** the outer ring (37) is supported in the cavity (13) free from clearance transversally to the central axis (10).

10. Device (14) according to one of the claims from 1 to 9, **characterized in that** a gasket (31) is arranged between the container (6) and the lid (8).

11. Device (14) according to claim 10, **characterized in that** the spring element (30) and the gasket (31) are configured in a single piece.

12. Device (14) according to claim 10 or 11, **characterized in that** the height H of the cavity (13) measured parallel to the central axis (10) is defined by the heights of the outer ring (37) and the gasket (31) measured parallel to the central axis (10).

13. Device (14) according to claim 10 or 11, **characterized in that** the height measured parallel to the central axis (10) is defined by internal elements of various heights, and preferably by the lid (8).

14. Device (14) according to one of the claims from 1 to 13, **characterized in that** the elastic means (19) have in an unloaded condition a height h measured parallel to the central axis (10) and are compressible in an axial direction by a measure Δh, and that the ratio Δh : h is between 0.001 % and 99.999 %, and preferably between 0.01 % and 99.99 %.

15. Device (14) according to claim 14, **characterized in that** the ratio Δh : h is between 0.1 % and 99.9 %, and preferably between 1 % and 99 %.

16. Device (14) according to claim 15, **characterized in that** the ratio Δh : h is between 5 % and 95 %, and preferably between 10 % and 90 %.

17. Device (14) according to one of the claims from 1 to 16, **characterized in that** the container (6) is configured in a circular cylindrical form, presents an internal thread (7) coaxially arranged in the cavity (13), and that the lid (8) comprises an external thread (9) complementary to the inner thread (7).

18. Device (14) according to on e of the claims from 1 to 17, **characterized in that** the container (6) contains a porous bone replacement material (1), whose total volume v is smaller than the volume V of the cavity (13).

19. Device (14) according to claim 18, **characterized in that** the bone replacement material (1) is present in the form of a block, preferably in the form of a cube, cylinder, hollow cylinder, disc, wedge, cone, truncated cone or a ball.

20. Device according to claim 18 or 19, **characterized in that** the bone replacement material (1) is accommodated inside an implant in such a way that it communicates with the surface of the implant at least in part.

21. Device according to claim 20, **characterized in that** the implant is chosen from one of the following groups of materials:
A) metal and/or
B) synthetic material and/or
C) non-metal, preferably glass and/or
D) ceramics of various densities, and/or
E) composite ceramics.

22. Device according to one of the claims from 1 to 21, **characterized in that** the elastic means (19) is arranged symmetrically to the central axis (10).

23. Device according to one of the claims from 1 to 21, **characterized in that** the elastic means (19) is arranged asymmetrically to the central axis (10).

24. Device according to one of the claims from 1 to 23, **characterized in that** the bottom (16) of the container (6) is fitted with a centrally perforated connecting piece (21), which forms one of the two openings (4).

25. Device according to one of the claims from 1 to 23, **characterized in that** the bottom (16) of the container (6) is fitted with a de-centrally perforated connecting piece (21), which forms one of the two openings (4).

26. Device according to one of the claims from 1 to 25, **characterized in that** a centrally or de-centrally perforated connecting piece, which forms one of the two openings (3), is arranged on the outside of the cover plate (18) of the lid (8).

27. Use of the device according to one of the claims from 1 to 26 for impregnating a porous bone replacement material (1) with an impregnation agent.

## Revendications

1. Dispositif (14) pour l'imprégnation d'un matériau poreux de substitution osseuse (1) avec un agent d'imprégnation (5), comprenant
A) un récipient (6) avec un couvercle (8), lequel récipient présente un axe central (10) et un espace creux (13) ; dans lequel
B) le récipient (2) présente deux ouvertures (3, 4) qui peuvent être fermées,
**caractérisé en ce que**
C) le récipient (6) comprend des moyens élastiques (19) disposés dans l'espace creux (13), au moyen desquels une force de serrage peut être exercée sur un matériau poreux de substitution osseuse (1) placé dans l'espace creux (13) ;
D) le récipient (6) est conçu pour pouvoir être mis sous vide ; et
E) le récipient (6), à l'intérieur de son espace creux (13), présente un taraudage (7) coaxial à l'axe central (10), si bien que le couvercle (8), lequel, sur sa face latérale, présente un filetage (9) complémentaire au taraudage (7), peut être relié de manière amovible avec le récipient (6).

2. Dispositif (14) selon la revendication 1, **caractérisé en ce que** les moyens élastiques (19) sont réalisés sous la forme d'un soufflet ou d'un ressort.

3. Dispositif (14) selon la revendication 1, **caractérisé en ce que** les moyens élastiques (19) comprennent un élément élastique (30) disposé coaxialement à l'axe central (10) présentant plusieurs barrettes élastiques (34) disposées en forme hélicoïdale ou spirale par rapport à l'axe central (10).

4. Dispositif (14) selon la revendication 3, **caractérisé en ce que** les barrettes (34) présentent des extrémités supérieures (42) dirigées vers le couvercle (8), qui débouchent dans une plaque centrale (41) pourvue de perforations (44) et disposée de manière concentrique à l'axe central (10), dont le diamètre d mesuré orthogonalement à l'axe central (10) est compris entre 0,001 % et 99,999%, de préférence entre 0,1% et 99,9% du diamètre D de l'espace creux (13) orthogonal à l'axe central (10).

5. Dispositif (14) selon la revendication 4, **caractérisé en ce que** le diamètre d est compris entre 1% et 99%, de préférence entre 5% et 95% du diamètre D.

6. Dispositif (14) selon la revendication 5, **caractérisé en ce que** le diamètre d est compris entre 10% et 90%, de préférence entre 20% et 80% du diamètre D.

7. Dispositif (14) selon la revendication 6, **caractérisé en ce que** le diamètre d est compris entre 25% et 75%, de préférence entre 30% et 40% du diamètre D.

8. Dispositif (14) selon l'une quelconque des revendications 3 à 7, **caractérisé en ce que** les barrettes (34) présentent des extrémités inférieures (43) qui débouchent dans un anneau externe (37) de l'élément élastique (30).

9. Dispositif (14) selon la revendication 8, **caractérisé en ce que** l'anneau externe (37) est logé sans jeu dans l'espace creux (13) transversalement à l'axe central (10).

10. Dispositif (14) selon l'une quelconque des revendications 1 à 9, **caractérisé en ce qu'**un joint d'étanchéité (31) est disposé entre le récipient (6) et le couvercle (8).

11. Dispositif (14) selon la revendication 10, **caractérisé en ce que** l'élément élastique (30) et le joint d'étanchéité (31) sont formés d'une seule pièce.

12. Dispositif (14) selon la revendication 10 ou 11, **caractérisé en ce que** la hauteur H de l'espace creux (13) mesurée parallèlement à l'axe central (10) est définie par les hauteurs de l'anneau externe (37) et du joint d'étanchéité (31) mesurées parallèlement à l'axe central (10).

13. Dispositif (14) selon la revendication 10 ou 11, **caractérisé en ce que** la hauteur mesurée parallèlement à l'axe central (10) est définie par des éléments internes de différentes hauteurs, de préférence par le couvercle (8).

14. Dispositif (14) selon l'une quelconque des revendications 1 à 13, **caractérisé en ce que** les moyens élastiques (19) présentent une hauteur h mesurée à l'état non sollicité parallèlement à l'axe central (10) et qu'ils peuvent être comprimés axialement selon une valeur Ah, et **en ce que** le rapport Δh:h est compris entre 0,001% et 99,999%, de préférence entre 0,01% et 99,99%.

15. Dispositif (14) selon la revendication 14, **caractérisé en ce que** le rapport Δh:h est compris entre 0,1% et 99,9%, de préférence entre 1% et 99%.

16. Dispositif (14) selon la revendication 15, **caractérisé en ce que** le rapport Δh:h est compris entre 5% et 95%, de préférence entre 10% et 90%.

17. Dispositif (14) selon l'une quelconque des revendications 1 à 16, **caractérisé en ce que** le récipient (6) est réalisé en forme de cylindre circulaire, qu'il présente un taraudage (7) disposé coaxialement à l'intérieur de l'espace creux (13) et que le couvercle (8) comprend un filetage (9) complémentaire au taraudage (7).

18. Dispositif (14) selon l'une quelconque des revendications 1 à 17, **caractérisé en ce que** le récipient (6) contient un matériau de substitution osseuse poreux (1), dont le volume total v est inférieur au volume V de l'espace creux (13).

19. Dispositif (14) selon la revendication 18, **caractérisé en ce que** le matériau de substitution osseuse (1) est présent sous la forme d'un bloc, de préférence sous la forme d'un cube, d'un cylindre, d'un cylindre creux, d'un disque, d'un coin, d'un cône, d'un tronc de cône ou d'une sphère.

20. Dispositif selon la revendication 18 ou 19, **caractérisé en ce que** le matériau de substitution osseuse (1) est logé dans un implant de telle sorte qu'il communique au moins en partie avec la surface de l'implant.

21. Dispositif selon la revendication 20, **caractérisé en ce que** l'implant est choisi dans un des groupes de matériaux suivants :
A) métal et/ou
B) matière plastique et/ou
C) non-métal, de préférence le verre et/ou
D) des céramiques de densités différentes et/ou
E) des céramiques composites.

22. Dispositif selon l'une quelconque des revendications 1 à 21, **caractérisé en ce que** les moyens élastiques (19) sont disposés de manière symétrique par rapport à l'axe central (10).

23. Dispositif selon l'une quelconque des revendications 1 à 21, **caractérisé en ce que** les moyens élastiques (19) sont disposés de manière asymétrique par rapport à l'axe central (10).

24. Dispositif selon l'une quelconque des revendications 1 à 23, **caractérisé en ce que** le fond (16) du récipient (6) est pourvu d'un élément de raccordement (21) centré alésé, lequel élément forme l'une des deux ouvertures (4).

25. Dispositif selon l'une quelconque des revendications 1 à 23, **caractérisé en ce que** le fond (16) du récipient (6) est pourvu d'un élément de raccordement (21) décentré alésé, lequel élément forme l'une des deux ouvertures (4).

26. Dispositif selon l'une quelconque des revendications 1 à 25, **caractérisé en ce qu'**un élément de raccordement (25), centré ou décentré, alésé, lequel forme l'une des deux ouvertures (3), est disposé à l'extérieur au niveau de la plaque de recouvrement (18) du couvercle (8).

27. Utilisation du dispositif selon l'une quelconque des revendications 1 à 26 pour l'imprégnation d'un matériau de substitution osseuse poreux (1) avec un agent d'imprégnation (5).
